(19) Europäisches Patentamt

European Patent Office

Office européen des brevets

(11) EP 0 737 690 A2

(12) EUROPEAN PATENT APPLICATION

(43) Date of publication:
16.10.1996 Bulletin 1996/42

(51) Int Cl.6: C07K 7/00

(21) Application number: 96302522.6

(22) Date of filing: 10.04.1996

(84) Designated Contracting States:
CH DE DK FR GB IT LI NL

(30) Priority: 10.04.1995 JP 84247/95

(71) Applicant: The Calpis Food Industry Co., Ltd.
Shibuya-ku Tokyo (JP)

(72) Inventors:
• Maeno, Masafumi
Machida-shi, Tokyo (JP)
• Maeno, Masafumi
Machida-shi, Tokyo (JP)

(74) Representative: Hallybone, Huw George
CARPMAELS AND RANSFORD
43 Bloomsbury Square
London WC1A 2RA (GB)

(54) Antihypertensive agent and method for preparing same

(57) An antihypertensive agent contains an effective amount of a peptide selected from a peptide having an amino acid sequence of Lys Val Leu Pro Val Pro Gln and a pharmaceutically acceptable salt thereof, a peptide having an amino acid sequence of Tyr Lys Val Pro Gln Leu and a pharmaceutically acceptable salt thereof, and mixtures thereof.

## Description

### BACKGROUND OF THE INVENTION

This invention relates to an antihypertensive agent containing a peptide or peptides and/or a salt thereof and having antihypertensive effect for medical use, foods for specified health use and healthy foods. The invention also relates to a method for preparing the antihypertensive agent.

Numerous reports have been made on natural and synthetic chemical products effective as antihypertensive agents. Among well-known synthetic chemical products is captopril (D-2-methyl-3-mercaptopropanoyl-L-proline) which has already been put to practical use as an orally administered antihypertensive agent. However, special attention must be paid at all times to safety aspects of these synthetic chemical products.

As antihypertensive natural products or products derived from natural products, venom of *Bothrops jararaca* and analogues thereof have been known. Reports have also been made that peptide substances derived from food materials exhibit hypertensive effects. For example, it has been reported that peptides obtained from enzymatically digested products of animal milk casein or fish meat protein exhibit antihypertensive effects. Recently, a report has been made on the antihypertensive effect of casein digested peptides by lactic acid bacteria producing proteinase (N. Yamamoto et al., J. Dairy Sci. 77: 917-922 (1994), N. Yamamoto et al., Biosci. Biotech. Biochem., 58,776-778 (1994)).

### SUMMARY OF THE INVENTION

It is a principal object of the present invention to provide an antihypertensive agent having high safety and exhibiting superior antihypertensive effect by oral administration of a small amount, and a method for preparing same.

According to the present invention, there is provided an antihypertensive agent including an effective amount of a peptide selected from the group consisting of a peptide having an amino acid sequence of Lys Val Leu Pro Val Pro Gln and a pharmaceutically acceptable salt thereof, a peptide having an amino acid sequence of Tyr Lys Val Pro Gln Leu and a pharmaceutically acceptable salt thereof, and mixtures thereof.

According to the present invention, there is also provided a method for preparing the above antihypertensive agent including fermenting an animal milk starting material with a microorganism.

According to the present invention, there is also provided a method for preparing the above antihypertensive agent including digesting an animal milk starting material with proteinase.

### BRIEF DESCRIPTION OF THE DRAWINGS

Fig.1 is a graph showing four aliquots A to D of a fraction in which antihypertensive effect was noticed in Example 1.

Fig.2 is a graph showing the relation between the changes in systolic blood pressure and the dose of Lys Val Leu Pro Val Pro Gln in Example 3.

### PREFERRED EMBODIMENTS OF THE INVENTION

The present invention will be explained in further detail hereinbelow.

The antihypertensive agent of the present invention contains an effective amount of a peptide or peptides represented by the above amino acid sequence or sequences and/or a salt thereof. The salt may be enumerated by a pharmaceutically acceptable salt, including inorganic acid salt, such as hydrochlorate, sulfate or phosphate, and organic acid salt, such as citrate, maleate, fumarate, tartarate or lactate.

The peptides may be prepared by fermenting an animal milk starting material with a microorganism, or digesting the starting material with proteinase, as later explained in detail and, if necessary, subsequently purifying the objective peptide or peptides by known technique. That is, each of the above-mentioned peptides is a peptide fragment of β-casein, which is a component of the animal milk protein. Each peptide may also be prepared by known methods of organic chemical synthesis. Since the antihypertensive agent of the present invention contains the peptide or peptides and/or a salt thereof, it is not absolutely necessary to carry out purification unless it is intended to isolate each of the peptides.

In the above method for fermentation, the animal milk starting material includes animal milk such as cow's milk, goat's milk or horse's milk; medium of animal skim milk; animal milk casein, animal milk caseinate; and BL broth (available from NISSUI PHARMACEUTICAL CO., LTD.), Briggs liver broth, Lactobacilli MRS broth (available from DIFCO LABORATORIES, USA) or GAM broth (available from NISSUI PHARMACEUTICAL CO., LTD.), to which are added animal milk casein and/or animal milk caseinate. The microorganism includes lactic acid bacteria, in particular lactic acid bacteria of the genus *Lactobacillus,* such as *Lactobacillus helveticus, Lactobacillus delbrueckii* subsp. *bulgaricus* or *Lactobacillus acidophilus,* preferably *Lactobacillus helveticus* JCM-1003, *Lactobacillus delbrueckii* subsp. *bulgaricus* JCM-1002 or *Lactobacillus acidophilus* JCM-1132. *Lactobacillus helveticus* JCM-1003, *Lactobacillus delgiueckii* subsp. *bulgaricus* JCM-1002 and *Lactobacillus acidophilus* JCM-1132 are all publicly known and listed in Japan Collection of Microorganisms, The Institute of Physical and Chemical Research of Wako, Saitama, Japan so that these strains are commercially available to the public.

The aforementioned animal milk starting material is desirably fermented at a fermentation temperature of 20° to 50°C, preferably 30° to 45°C for a fermentation time duration of 3 to 30 hours and preferably 6 to 12 hours. Although the fermentation time duration differs depending upon the inoculum size of a starter culture and the fermentation temperature, the fermented milk containing the targeted peptide or peptides may be produced by carrying out the fermentation until the amount of fermented lactic acid in the fermented milk amounts to preferably to 0.5 to 2.0 wt% and more preferably to 0.7 to 1.5 wt%. Specifically, the amount of fermented lactic acid may be set to 0.7 to 0.8 wt% with the inoculum size of the starter culture of 3 wt%, the fermentation temperature of 37°C and the fermentation time duration of approximately 8 hours. If the inoculum size of the starter culture is 1 wt% and the fermentation temperature is 37°C, the amount of fermented lactic acid may be 0.7 to 0.8 wt% in the fermentation time duration on the order of 10 to 12 hours. If fermentation is carried out for longer time, the amount of the targeted peptide or peptides is decreased since the peptide or peptides are susceptible to the action of peptidase produced by the lactic acid bacteria. If fermentation is continued further, Val Pro Pro or Ile Pro Pro is newly produced under the action of the peptidase to exhibit antihypertensive effect (Journal of Japan Society for Bioscience, Biotechnology and Agrochemistry, 67, 3, 289 (1993), J. of Dairy Sci., 78, 777-783 (1995) and J. of Dairy Sci., 78, 253-1257 (1995)). However, under the above fermentation conditions, the production of Val Pro Pro or Ile Pro Pro in an amount sufficient to attain the antihypertensive effect is not noticed.

For digesting the aforementioned animal milk starting material with proteinase, animal milk casein or animal milk caseinate may be used as an animal milk starting material and a microorganism producing proteinase, for example, may be employed as proteinase.

The microorganism producing proteinase from a microorganism, such as lactic acid bacteria, may be employed. The method of digesting casein purified by purified lactic acid bacteria producing proteinase has so far been known. For example, although a report has been made on the properties of *Lactobacillus helveticus* producing proteinase and on the cleavage site of an amino acid sequence of casein in Claude Zevaco and J.C. Gripon, Le Lait, 68, 393-408 (1988) and Yamamoto N. et al., J. Biochem., 114, 740-745 (1993), no report has been made of cleavage to give the peptide of Lys Val Leu Pro Val Pro Gln or the peptide of Tyr Lys Val Pro Gln Leu. In addition, although a report has been made on comparison of purified β-casein hydrolysate by *Lactobacillus lactis* subsp. *cremoris* producing P1 and P3 type purified proteinase in Julian R. Reid et al. Applied Microbiology and Biotechnology, 36, 344-351 (1991), there is no description on the practical production of the above-mentioned peptides according to the present invention. In addition, no report has been made on the antihypertensive effect by the above-mentioned peptides nor a method of producing the peptides from the fermented milk.

The lactic acid bacteria for preparing lactic acid bacteria producing proteinase and medium for cultivating the bacteria may be the same as those enumerated in connection with the fermentation method.

The above-mentioned proteinase may be produced at a fermentation temperature of 20° to 50°C, preferably 30° to 45°C and for a fermentation time duration of 3 to 30 hours, preferably 6 to 12 hours. The produced culture broth usually has a pH value of 3 to 4. For raising the yield of the targeted proteinase, it is desirable to maintain the culture broth in a neutral pH range, that is in a pH range of 5 to 8. The proteinase may be extracted by repeating the extraction process continuing preferably for 10 to 60 minutes at 5° to 40°C two to five times.

For digesting the animal milk starting material with the above-mentioned lactic acid bacteria producing proteinase, the lactic acid bacteria producing proteinase is mixed with the animal milk starting material dissolved in a buffer, such as phosphoric acid buffer in order to carry out reaction at 30° to 50°C for one to 12 hours followed by purification. The resulting reaction liquid is subjected to centrifugal separation and ultrafiltration through an ultrafiltration membrane, preferably with a molecular weight fractionation of 10000 to 50000. The permeated solution is purified with reverse phase high performance liquid chromatography to give a component containing the desired peptide or peptides. The weight ratio of the lactic acid bacteria producing proteinase to the animal milk starting material is preferably 1:100 to 1:5000.

Since the antihypertensive agent according to the present invention contains the above-mentioned peptide or peptides and/or a salt thereof, it may be effectively employed for therapy and prevention of hypertension in mammals including human being.

The antihypertensive agent of the present invention may be administered orally or intravenously. The dosage form may be liquid or solid. In the latter case, the agent is powdered and formed into tablets, granules or capsules. The peptides and salts thereof may be used as they are produced by fermentation, enzymatic digestion or organic synthesis methods, or formed into the above dosage form subsequent to purification by known purification methods. Since the peptides and the salt thereof exhibit antihypertensive effects if used in minor quantities, as later explained, the solution containing the peptides and salts thereof obtained by the enzymatic digestion or fermentation may be used directly or in conjunction with various nutrients. Alternatively, the solution may be contained in foods or beverages so as to be used as functional foods, foods for specified health use or healthy foods having the antihypertensive effects and the function of preventing hypertension. The fermented product may be used as fermented food for direct intake, such as yogurt or cheese.

The dosage of the antihypertensive agent according to the present invention is not less than 0.2 mg/kg of body weight/day, preferably 0.2 mg/kg of body weight/day to 10.0 mg/kg of body weight/day and more preferably approximately 1 mg/kg of body weight/day, depending on the age, body weight and symptoms of hypertension. If the fermented milk obtained by the fermentation method is ingested as yogurt, the targeted antihypertensive effect may be acquired with the amount of intake of preferably 5 to 20 ml/kg of body weight/day.

The above-mentioned peptides were orally administered to WKY rats (Wister Kyoto rats; 15 weeks of age with body weight of approximately 300 g, n=5) in amounts of 10 mg/kg of body weight/day for one month. It was found that the animals showed no unusual states, thus demonstrating safety of the respective peptides.

The antihypertensive agent of the present invention may be utilized for medicines or functional foods since it is capable of effectively lowering the blood pressure of human being and mammals while being high in safety and relatively free from side effects. The antihypertensive agent may be easily and inexpensively prepared by fermenting animal milk or digesting the animal milk with proteinase.

## EXAMPLES OF THE INVENTION

The present invention will be explained in further detail with reference to Examples which are given only for illustration and are not intended for limiting the invention.

## Example 1

10 kg of 9 wt% skim milk powders were inoculated with 300 g of a starter culture obtained by fermenting *Lactobacillus helveticus* JCM-1003 as lactic acid bacteria. To the resulting product was automatically added a 3N solution of sodium hydroxide and the resulting product was cultured at 37°C for 5 hours, as pH was maintained at 6.0 until turbidity of 1.0 (O.D.$_{590}$) was reached. For measuring turbidity, sodium citrate was added to 10 ml of the fermented culture so that the ultimate concentration reached 1 wt% for solubilizing milk protein and absorbance at 590 nm was measured. After the end of cultivation, sodium citrate was added to the fermented culture so that the amount of sodium citrate amounted to 1 wt%. The resulting product was held at room temperature for 20 minutes for solubilizing the milk protein. The resulting product was subjected to centrifugal separation for 20 minutes at 5000 rpm for collecting lactic acid bacteria. The lactic acid bacteria thus collected were washed with a buffer (pH8) containing 20 mM calcium chloride and 50 mM β-sodium glycerophosphate and admixed with 50 ml of 50 mM tris-hydrochloric acid buffer (pH8.0). The resulting mixture was kept at 37°C for 30 minutes and extracted. The resulting solution was subjected to centrifugal separation at 10000 rpm for ten minutes for sampling a supernatant. The extraction and centrifugal separation were repeated four times for sampling 200 ml of the supernatant (crude extractant). The crude extractant was passed through a DEAE-Sepharose column (5 ml capacity) previously equilibrated with a 20 mM tris-hydrochloric acid buffer, pH8.0 (TE buffer) containing 5 mM of ethylene diamine tetraacetic acid (EDTA). The column was washed with 30 ml of a TE buffer containing 0.3 M of sodium hydrochloride and eluted with a TE buffer containing 1.0 M sodium chloride for producing approximately 150 µg of substantially pure lactic acid producing proteinase.

1 g of casein was dissolved in 100 ml of a 20 mM phosphoric acid buffer (pH 7.5) and mixed with 50 µg of the lactic acid bacteria producing proteinase. The reaction proceeded at 42°C for 15 hours. The reaction liquid was subjected to centrifugal separation at 10000 rpm for 10 minutes. After the centrifugal separation, a supernatant was recovered and filtered using an apparatus "ADVANTEC TOYO UHP-150" manufactured by TOYO ROSHI CO., LTD. with an ultrafiltration membrane with a molecular weight fractionation of 10000 manufactured by FUJI FILTER LTD., Tokyo, Japan. About 700 mg of proteinase digested peptide could be produced from 1 g of casein in 90 ml of an eluent obtained by ultrafiltration with a yield of approximately 70%. The eluent obtained by ultrafiltration was fractionated every ten minutes, using reverse phase high performance liquid chromatography (HPLC) under the conditions of:

Pump, L6200 INTELLIGENT pump manufactured by HITACHI LTD., and L6000 pump manufactured by HITACHI LTD.;
Detector, L4000 UV detector manufactured by HITACHI LTD.;
215 nm UV absorbing column, WATERS µ-BONDANSPHERE 5µC18 manufactured by NIHON MILLIPORE LTD., Tokyo, Japan;
Eluent, A-liquid (0.1 wt% aqueous solution of trifluoroacetic acid (TFA)); and B-liquid, 0.1 wt% TFA containing acetonitrile;
Gradient, (B-liquid/A-liquid + B-liquid) × 100(%): 0% → 40% (60 minutes); abbreviated hereinafter to gradient (1);

flow rate, 1 ml/min, in order to produce six eluted fractions.

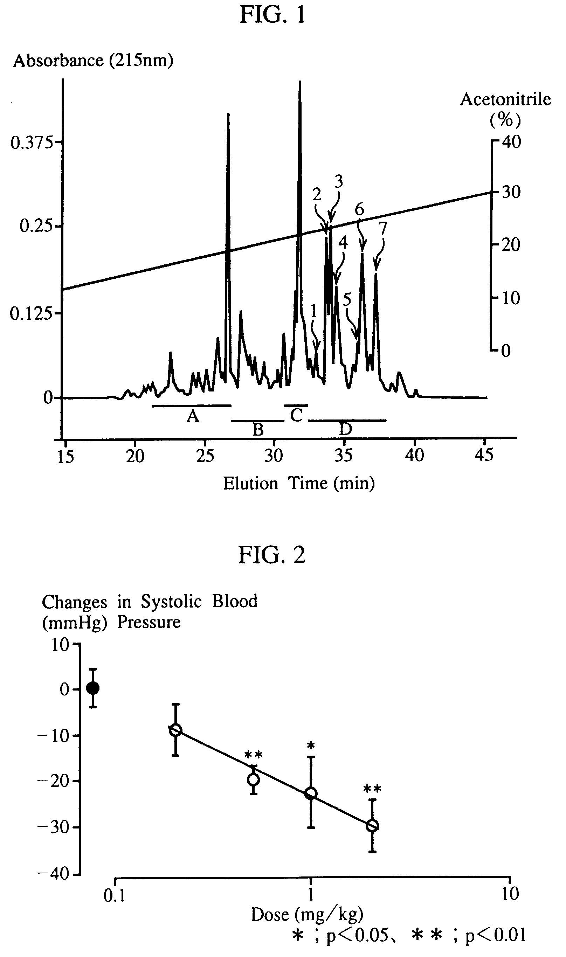

The eluted fractions were freeze-dried and dissolved in 50 ml of phosphate buffered saline (PBS). These fractions were forcedly administered to spontaneously hypertensive rats (SHR; purchased from CHARLES RIVER JAPAN INC., five rats per group, 19 to 25 weeks of age, male) by a stomachic sonde in each amount of 2 ml per rat. The changes in systolic blood pressure were compared to a control group (0.05% casein in PBS) after 6 hours. The blood pressure was measured, using a programmed electrosphygmomanometer "PE-300" manufactured by NARCO BIO-SYSTEMS CO., LTD. and the systolic blood pressure was measured by the tail cuff method. Since the significant antihypertensive effect was observed in a fraction sampled after 20 to 30 minuets after the start of elution, 25 ml of this fraction was again fractionated by HPLC by the above method to obtain the results of elution of Fig.1. Four fractions A, B, C and D were sampled. The total amounts of the eluted fractions were freeze-dried and dissolved each in 25 ml of phosphate buffered saline. The resulting solution was forcedly administered to spontaneously hypertensive rats in accordance with the above method and the changes in systolic blood pressure after six hours were measured. Since the significant decrease in the systolic blood pressure was found in the fraction D, 10 ml of this fraction D were further fractionated by HPLC by the above method and 7 main peaks were sampled. The peaks of numbers 1 to 7 in Fig.1 contain the following peptides:

1. Peak containing peptide of Lys Val Leu Pro Val Pro Gln
2. Peak containing peptide of Leu Gln Ser Trp
3. Peak containing peptide of Arg Glu Leu Glu Glu Leu
4. Peak containing peptide of Thr Lys Val Ile Pro
5. Peak containing peptide of Ala Met Lys Pro Trp
6. Peak containing peptide of Tyr Lys Val Pro Glu Leu, peptide of Met Lys Pro Trp Ile Gln Pro Lys and peptide of Leu Leu Tyr Gln Gln Pro Val
7. Peak containing peptide of Ala Tyr Phe Tyr Pro and peptide of Ala Met Lys Pro Trp Ile Gln Pro Lys

The total amounts of sampled peaks were purified using HPLC under the same conditions as those of the above method except that the peaks were freeze-dried and dissolved in 5 ml of water and the gradient was changed in accordance with the following:

$$(B\text{-liquid}/A\text{-liquid} + B\text{-liquid}) \times 100(\%):15\% \rightarrow 30\% \ (40 \text{ minutes})$$

This gradient is referred to hereinafter as the gradient (2).

The ultimately purified fractions were freeze-dried and dissolved in 30 $\mu$l of water. The resulting solutions were analyzed as for the amino acid sequences from the N terminals of the peptides by Automatic Protein Sequencer PPSQ-10 SYSTEM manufactured by SHIMADZU CO. 10 types of peptides were thus identified. Table 1 shows the amino acid sequences of the peptides.

TABLE 1

| | | | | | | | | | |
|---|---|---|---|---|---|---|---|---|---|
| Peptides Produced by Proteinase | | | | | | | | | |
| 1) | Lys | Val | Leu | Pro | Val | Pro | Gln | | |
| 2) | Leu | Gln | Ser | Trp | | | | | |
| 3) | Arg | Glu | Leu | Glu | Glu | Leu | | | |
| 4) | Thr | Lys | Val | Ile | Pro | | | | |
| 5) | Ala | Met | Lys | Pro | Trp | | | | |
| 6) | Tyr | Lys | Val | Pro | Gln | Leu | | | |
| 7) | Met | Lys | Pro | Trp | Ile | Gln | Pro | Lys | |
| 8) | Leu | Leu | Tyr | Gln | Gln | Pro | Val | | |
| 9) | Ala | Tyr | Phe | Tyr | Pro | | | | |
| 10) | Ala | Met | Lys | Pro | Trp | Ile | Gln | Pro | Lys |

## Example 2

The peptides having the same amino acid sequences as the ten peptides obtained in Example 1 were synthesized in accordance with the solid phase method using an automatic peptide synthesizer PPSM-8 manufactured by SHIMADZU CO. The antihypertensive effect of each synthesized peptide was measured by the following method. That is, 1 mg/kg of body weight of each peptide was forcedly administered by a stomachic sonde and comparison was made with the control group. The animals and the method of measuring the blood pressure were the same as in Example 1. Two types of peptides Lys Val Leu Pro Val Pro Gln and Tyr Lys Val Pro Gln Leu were found to exhibit significant antihypertensive effect. A significant difference with respect to the control group was noted by the fact that the values of systolic blood pressure change were -24.1±7.8* and -12.5±11.1**, thus showing a significant difference with respect to the control group (*p<0.05; **p<0.1). The results are shown in Table 2 in terms of (systolic blood pressure value before administration - systolic blood pressure value after administration)±SE.

TABLE 2

| Changes in Systolic Blood Pressure of Peptides | | |
|---|---|---|
| Amino Acid Sequence | | Changes in Systolic Blood Pressure Dose 1mg/kg (mmHg) |
| 1) | Lys Val Leu Pro Val Pro Gln | -24±7.8* |
| 2) | Leu Gln Ser Trp | -1.6±9.6 |
| 3) | Arg Glu Leu Glu Glu Leu | 1.3±6.7 |
| 4) | Thr Lys Val Ile Pro | -9.2±6.1 |
| 5) | Ala Met Lys Pro Trp | -4.6±11.6 |
| 6) | Tyr Lys Val Pro Gln Leu | -12.5±11.1** |
| 7) | Met Lys Pro Trp Ile Gln Pro Lys | -2.9±5.9 |
| 8) | Leu Leu Tyr Gln Gln Pro Val | 1.2±9.8 |
| 9) | Ala Tyr Phe Tyr Pro | -4.3±6.6 |
| 10) | Ala Met Lys Pro Trp Ile Gln Pro Lys | -0.6±9.3 |

*; P<0.05
**; P<0.1

Example 3

Of one of the two peptides which demonstrated significant blood pressure decrease in Example 2, that is the peptide of Lys Val Leu Pro Val Pro Gln, dose response concerning the amount of administration and the degree of blood pressure decrease was investigated. The animals and the method of measuring blood pressure were the same as those of Example 1. Specifically, blood pressure was measured by orally administering 0.2, 0.5, 1.0 and 2.0 mg of the peptide per kg of body weight to rats of the respective groups and the systolic blood pressure after lapse of six hours as from administration was measured and compared to the corresponding values of the control group. The results are shown in Fig.2, from which it is seen that dosage-dependent effects were noticed for the range of 0.5 to 2.0 mg/kg of body weight.

Example 4

Measurement was made of two types of peptides (Lys Val Leu Pro Val Pro Gln and Tyr Lys Val Pro Gln Leu) contained in fermented milk.

*Lactobacillus helveticus* JCM-1003 was used as lactic acid bacteria. 15 g of a 9 wt% skim milk medium were inoculated with the lactic acid bacteria and cultured for 20 hours. 500 g of a further 9 wt% skim milk medium were inoculated with the total quantity of the culture and cultivation was continued at 37°C until the quantity of lactic acid reached 0.8% to produce yogurt like products. The yogurt like products were liquefied by a homogenizer manufactured by MITAMURA RIKEN KOGYO LTD. under the trade name of D7801 and subjected to centrifugal separation for ten minutes at 10000 rpm. 100 g of the produced supernatant was filtered by ultrafiltration using an apparatus manufactured by TOYO ROSHI CO., LTD. under the trade name of ADVANTEC TOYO UHP-150 with an ultrafiltration membrane of molecular weight fractionation of 10000 manufactured by FUJI FILTER LTD., to produce an eluent obtained by ultrafiltration. After freeze-drying, 3 ml of the thus obtained eluent was dissolved in 1 ml of water and the total quantity thereof was analyzed in accordance with the gradient (1) by HPLC under the elution conditions of Example 1. The peaks eluted during the same elution time as that in which the peptides were produced in Example 1 in accordance with the gradient (1) were sampled. The elution time was 17.3 minutes for Lys Val Leu Pro Val Pro Gln and 23.0 minutes for Tyr Lys Val Pro Gln Leu.

The total quantity of the fraction thus obtained was freeze-dried and dissolved in 1 ml of water and analyzed by HPLC in accordance with the gradient (2) under the elution conditions of Example 1. The peaks eluted during the same elution time as that in which the peptides were produced in Example 1 in accordance with the gradient (2) were sampled. The elution time was 7.1 minutes for Lys Val Leu Pro Val Pro Gln and 17.0 minutes for Tyr Lys Val Pro Gln Leu. The total quantity of the produced fractions were freeze-dried and dissolved in 30 μl of water and analyzed by the automatic protein sequencer PPSQ-10 manufactured by SHIMADZU CO. beginning from the N terminals of the peptides. The peptides were identified as Lys Val Leu Pro Val Pro Gln and Tyr Lys Val Pro Gln Leu. Approximately 25 μg/ml of Lys Val Leu Pro Val Pro Gln and approximately 35 μg/ml of Tyr Lys Val Pro Gln Leu were contained in 500 g of the fermented milk.

Example 5

Using *Lactobacillus helveticus* JCM-1003, yogurt like products were produced in the same way as in Example 4. The yogurt like products were liquefied by a homogenizer manufactured by MITAMURA RIKEN KOGYO LTD. under the trade name of D7801 and 7 g of the yogurt like products per kg of body weight were forcedly administered to SHR using a stomachic sonde. The systolic blood pressure value after 6 hours was measured and compared to that of the group administered with non-fermented milk (9 wt% skim milk). The animal used and the method for measuring the blood pressure were the same as in Example 1. The results are shown in Table 3 in terms of [(systolic blood pressure value before administration) - (systolic blood pressure value after administration)] ± SE. It is seen from Table 3 that blood pressure was decreased significantly as compared to the group administered with non-fermented milk (-25.4±5.6∗∗; ∗∗$P < 0.01$).

TABLE 3

| Changes in Systolic Blood Pressure of Fermented Milk (Yogurt like products) | |
|---|---|
| Samples | Changes in systolic blood pressure (mmHg) |
| Fermented milk<br>Non-fermented milk | -25.4±5.6∗∗<br>-2.7±3.5 |

∗∗; $P < 0.01$

EP 0 737 690 A2

**Claims**

1. An antihypertensive agent comprising an effective amount of a peptide selected from a peptide having an amino acid sequence of Lys Val Leu Pro Val Pro Gln and a pharmaceutically acceptable salt thereof, a peptide having an amino acid sequence of Tyr Lys Val Pro Gln Leu and a pharmaceutically acceptable salt thereof, and mixtures thereof.

2. The antihypertensive agent according to claim 1 wherein the effective amount is 0.2 mg/kg of body weight/day to 10.0 mg/kg of body weight/day.

3. A method for preparing the antihypertensive agent claimed in claim 1 comprising fermenting an animal milk starting material with a microorganism.

4. The method for preparing the antihypertensive agent according to claim 3 wherein said microorganism is lactic acid bacteria.

5. The method for preparing the antihypertensive agent according to claim 4 wherein said lactic acid bacteria is lactic acid bacteria of the genus *Lactobacillus.*

6. The method for preparing the antihypertensive agent according to claim 5 wherein said lactic acid bacteria of the genus *Lactobacillus* is selected from *Lactobacillus helveticus, Lactobacillus delbrueckii* subsp. *bulgaricus, Lactobacillus acidophilus* and mixtures thereof.

7. The method for preparing the antihypertensive agent according to claim 3 wherein said animal milk starting material is fermented at a fermentation temperature of 20 to 50°C for a fermentation time of 3 to 30 hours.

8. A method for preparing the antihypertensive agent claimed in claim 1 comprising digesting an animal milk starting material with proteinase.

9. The method for preparing the antihypertensive agent according to claim 8 wherein said proteinase is produced by lactic acid bacteria.

10. The method for preparing the antihypertensive agent according to claim 9 wherein said lactic acid bacteria is lactic acid bacteria of the genus *Lactobacillus.*

11. The method for preparing the antihypertensive agent according to claim 10 wherein said lactic acid bacteria of the genus *Lactobacillus* is selected from *Lactobacillus helveticus, Lactobacillus delbrueckii* subsp. *bulgaricus, Lactobacillus acidophilus* and mixtures thereof.

12. The method for preparing the antihypertensive agent according to claim 9 wherein said proteinase is lactic acid bacteria producing proteinase and wherein said lactic acid bacteria producing proteinase is fermented at a fermentation temperature of 20 to 50°C for a fermentation time of 3 to 30 hours.

13. An agent according to claim 1 or claim 2 for use as an antihypertensive agent.

14. Use of an agent according to claim 1 or claim 2 in the manufacture of a medicament for use as an antihypertensive agent.

# FIG. 1

# FIG. 2

SEQUENCE LISTING

(1) GENERAL INFORMATION:

  (i) APPLICANT:
      (A) NAME: THE CALPIS FOOD INDUSTRY CO.,LTD.
      (B) STREET: 20-3 EBISU-NISHI, 2-CHOME
      (C) CITY: SHIBUYA-KU
      (D) STATE: TOKYO
      (E) COUNTRY: JAPAN
      (F) POSTAL CODE (ZIP): TOKYO

  (ii) TITLE OF INVENTION: ANTIHYPERTENSIVE AGENT AND METHOD FOR
           PREPARING THE SAME

  (iii) NUMBER OF SEQUENCES: 2

  (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)

(2) INFORMATION FOR SEQ ID NO: 1:

  (i) SEQUENCE CHARACTERISTICS:
      (A) LENGTH: 7 amino acids
      (B) TYPE: amino acid
      (C) STRANDEDNESS: single
      (D) TOPOLOGY: linear

  (ii) MOLECULE TYPE: peptide

  (xi) SEQUENCE DESCRIPTION: SEQ ID NO: 1:

  Lys Val Leu Pro Val Pro Gln
  1               5

(2) INFORMATION FOR SEQ ID NO: 2:

  (i) SEQUENCE CHARACTERISTICS:
      (A) LENGTH: 6 amino acids
      (B) TYPE: amino acid
      (C) STRANDEDNESS: single
      (D) TOPOLOGY: linear

  (ii) MOLECULE TYPE: peptide

  (xi) SEQUENCE DESCRIPTION: SEQ ID NO: 2:

  Tyr Lys Val Pro Gln Leu
  1               5

8